(19) Europäisches Patentamt
European Patent Office
Office européen des brevets

(11) **EP 3 998 032 B1**

(12) **EUROPEAN PATENT SPECIFICATION**

(45) Date of publication and mention
of the grant of the patent:
**11.09.2024 Bulletin 2024/37**

(21) Application number: **20837136.9**

(22) Date of filing: **01.06.2020**

(51) International Patent Classification (IPC):
**A61B 18/12** *(2006.01)*    **A61B 18/08** *(2006.01)*
**A61B 18/10** *(2006.01)*

(52) Cooperative Patent Classification (CPC):
**A61B 18/082; A61B 18/08; A61B 18/10;**
A61B 2017/00123; A61B 2018/00577;
A61B 2018/00642; A61B 2018/00672;
A61B 2018/00678; A61B 2018/00702;
A61B 2018/00732; A61B 2018/00827;
A61B 2018/00869; A61B 2018/00875;
A61B 2018/00892; A61B 2018/00988;    (Cont.)

(86) International application number:
**PCT/CN2020/093767**

(87) International publication number:
**WO 2021/004196 (14.01.2021 Gazette 2021/02)**

(54) **RADIO FREQUENCY THERMAL ABLATION SYSTEM**

HOCHFREQUENZWÄRMEABLATIONSSYSTEM

SYSTÈME D'ABLATION THERMIQUE RADIOFRÉQUENCE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.07.2019   CN 201910611775
16.09.2019   CN 201910796082**

(43) Date of publication of application:
**18.05.2022   Bulletin 2022/20**

(73) Proprietor: **Resonant Medical Technology Co.,
Ltd.
Suzhou, Jiangsu 215341 (CN)**

(72) Inventor: **TAN, Wei
Suzhou, Jiangsu 215341 (CN)**

(74) Representative: **Keil & Schaafhausen
Patentanwälte PartGmbB
Bockenheimer Landstraße 25
60325 Frankfurt am Main (DE)**

(56) References cited:
**EP-A1- 3 093 626        EP-A2- 1 906 853
CN-A- 101 505 672      CN-A- 101 862 219
CN-A- 105 496 548      CN-A- 107 317 484
US-A- 4 094 320          US-A- 5 152 762
US-A1- 2017 238 991**

(52) Cooperative Patent Classification (CPC): (Cont.)
     A61B 2090/065

**Description**

**Technical Field**

This application generally relates to ablation technology, and more particularly, to radio frequency thermal ablation technology.

**Background Art**

Radio frequency ablation is a widely used minimally invasive technology. In recent years, radio frequency ablation has been applied to a variety of diseases such as arrhythmia, cancer tumors, and even skin defects. With radio frequency thermal ablation technology radio frequency energy is not directly applied onto the human body, but is converted into heat energy inside the device, and then transferred to the human body to produce thermal ablation effects. Because this technology has less trauma to the human body and the ablation method can be precisely controlled by an electronic system, it has become a technology that is commonly used in powered minimally invasive surgery. For example, radio frequency ablation catheters are currently used to ablate bronchial nerves to treat asthma, to ablate prostate tissue through the urethra to treat enlarged prostate, to ablate peripheral blood vessels to shrink them, to ablate surface tissues of the esophagus to treat esophageal cancer, and to ablate surface tissues of uterus to treat uterine cancer, etc. These organs of the human body, such as blood vessels, bronchi, urethra, esophagus, uterus, etc., are basically slender and tubular structures, and they have similar requirements on the appearance, function and performance of a radio frequency catheter.

At present, vascular thermal ablation technology relies on adjusting the heating time at a certain temperature to control the degree of vasoconstriction. Radio frequency thermal ablation probes mostly use closed and insulated resistive heating elements, such as patents US7837677, EP1906853, US2017/0202600A1, EP2662044, and US10357305. These methods do not create an electric current circulation into human tissue. Therefore, the common practice in this field is to ignore the influence of external tissues on the characteristics of resistive windings and only rely on simple temperature feedback control for treatment.

US2017/238991A1 describes an electrosurgical element with an electrically conductive sheet is laminated to an electrically insulative sheet. An electrode is formed on the electrically conductive sheet. An electrically insulative layer is formed on a tissue contacting surface of the electrode. The individual electrodes are separated from the laminated electrically insulative sheet and the electrically conductive sheet. In another method, a flexible circuit is vacuum formed to create a desired profile. The vacuum formed flexible circuit is trimmed. The trimmed vacuum formed flexible circuit is attached to a jaw member of a clamp jaw assembly.

EP3093626A1 relates to a telemetry system for the wireless measurement of a quantity, comprising at least a passive transducer sensitive to a variation of said quantity and a reading unit coupled to said passive transducer and suitable to detect, condition and process information of a measurement coming from the passive transducer. Each passive transducer comprises a resistive transduction sensitive element, a calibration capacitor, suitable to define a working frequency of the system, and a first coupling inductor. The reading unit comprises a second coupling inductor suitable to realize an inductive coupling with said first coupling inductor, and detection means suitable to detect a variation of amplitude of the real part of the impedance of the assembly constituted by the passive transducer and the second coupling inductor, at the working frequency, said amplitude of the real part of the impedance depending on the resistance value of the sensitive element.

However, simply controlling the temperature and time cannot achieve desired effects with radio frequency thermal ablation. For example, the area heated by thermal ablation is generally not just a point, but an area. Limited by engineering feasibility and cost constraints, it is generally impossible to arrange dense temperature sensing points in the entire ablation area, which may cause the temperature of the local treatment area to deviate from the target temperature. The most typical failure is that the organ wall is tightly attached to the heating winding surface due to accidental high temperature when the sensing means are insufficient, and the treatment continues due to the lack of effective sensing means, resulting in stickiness and even carbonization, and even medical accidents.

**Content of Invention**

The purpose of this application is to provide a radio frequency thermal ablation system, which can use the parasitic capacitance between turns of the closed winding to monitor the state of the aperture shrinkage of the tubular organ during the thermal ablation process, and then configure the monitoring by designing the winding sensitivity operation point, enhance the sensitivity of detecting the inter-turn winding parasitic capacitance (WPC), thereby effectively control the treatment process and make key treatment parameters such as the distance of the organ tube wall controllable in the thermal ablation process. Eventually the system help to achieve safer and more accurate treatment.

This invention discloses a radio frequency thermal ablation system comprising:

a thermal ablation catheter that comprises an inductive insulated heating coil, wherein the inductance is designed to configure the operating frequency when detecting the parasitic capacitance between winding turns, and enhance the detecting sensitivity;

a radio frequency generator that supplies radio frequency power to heat said heating coil;

a WPC (winding parasitic capacitance) detecting device that monitors the change of the WPC between the insulation heating windings caused by the tubular organ wall approaching the insulated heating windings and/or the coagulation of body fluid during the heating process;

a control device that controls the amount of radio frequency power output from said radio frequency generator to said heating coil according to the change of said WPC between the windings detected by said WPC detecting device.

In line with the invention, said WPC detecting device monitors said change of WPC by monitoring the vector voltage and current on the said heating coil.

In one of the preferred embodiments, said control device computes the WPC value based on the detected vector voltage and current of said heating coil, and extracting the impedance phase of the WPC combined with the coil inductance; if said phase is within a specified range, said control method stops said radio frequency generator from outputting radio frequency power.

In one of the preferred embodiments, the output frequency of said control device is continuously adjustable, and said control device searches and adjusts the frequency of the radio frequency power output from said radio frequency generator in a preset frequency range, so that the difference between said frequency and the resonant frequency of said complex impedance is less than a preset threshold.

In one of the preferred embodiments, the operating frequency range includes the frequency range in which the phase of the complex impedance of the heating coil shifts from -90 degrees to 90 degrees after said parasitic capacitance is cancelled.

In one of the preferred embodiments, said inter-turn spacing of the heating coil satisfies the following condition: the resonance frequency caused by the inductance of said heating coil and expected WPC is within said frequency range, wherein said expected WPC is the inter-turn parasitic capacitance of said heating coil when the distance between blood vessel tissue and said thermal ablation catheter is within a pre-set range.

In one of the preferred embodiments, said radio frequency thermal ablation system comprises flash memory for storing pre-calibrated resonant frequency that is readable as the target resonant frequency to said control device.

A control method of a thermal ablation system is disclosed (not being part of the invention) that comprises said thermal ablation catheter that further comprises an insulated inductive heating coil; said control method configures the operating frequency, and enhances the sensitivity of WPC detection, based on the inductivity of the heating coil;

said thermal ablation system also comprises said radio frequency generator that generates radio frequency power to the insulated heating coil;

said control method comprises:

outputing heating radio frequency power to said insulated heating coil by said radio frequency generator,

monitoring the change of WPC caused by the tubular organ wall approaching the insulated heating windings and/or the coagulation of body fluid during the heating process,

and controlling the output radio frequency power from said radio frequency generator, based on the detected change in WPC.

Said monitoring the change of WPC further may comprise: monitoring said change of WPC by monitoring the vector voltage and current on the said heating coil; said method of controlling the output radio frequency power from said radio frequency generator, based on the detected change in WPC, which further comprises: computing the WPC value based on the detected vector voltage and current of said heating coil, and extracting the impedance phase of the WPC combined with the coil inductance; if said phase is within a specified range, said control method stops said radio frequency generator from outputting radio frequency power.

After said monitoring the complex impedance of said insulated heating coil in heating, said control method may further comprise:

in the pre-set operating frequency range, searching and adjusting the output frequency from said radio frequency generator, reducing the difference between said operating frequency and the resonant frequency of said complex impedance to be less than a pre-set threshold.

In the implementation of this disclosure, considering the variation of the tubular organ and adjacent tissue during the thermal ablation, the first step is to design an inductive insulated heating coil based on the WPC variation range during treatment. This inductive insulated heating coil is wound to centralize the inductance, where the inductance of the insulated heating coil is centralized instead of separated. The centralized design enables focused sensing while heating.

During the thermal ablation, parasitic capacitance (WPC) exists due to the wall of the tubular organ and the coagulating blood liquid. Generally, as the tubular organ shrinks and the body liquid further coagulating, the WPC gradually increases to resonate with the inductance, and the phase variation of the overall complex impedance with respect to frequency increases. This boosts the sensitivity of detecting the phase of the overall complex impedance, and therefore boosts the sensitivity of deriving WPC based on the impedance phase.

In the implementation of this disclosure, by adding in inductance in the insulated heating coil, the capability of measuring the WPC in real time is enhanced. Therefore, it can be more accurately monitored and controlled WPC, and then detected and controlled the degree of organ wall approaching the thermal ablation catheter or the degree of surrounding body fluid coagulation based on monitoring the change of complex impedance of said inductive insulated heating coil in the thermal ablation treatment process. The thermal ablation treatment process can be controlled and optimized according to the monitoring results. For example, when the complex impedance is close to the preset value, it can be judged that the tubular organ wall atrophy closing to the ablation catheter, and the control device can reduce the power output and the shrinkage of the wall, so as to control the treatment effect more smoothly and accurately.

Even further, because the sensing circuit performs with better sensitivity when the phase is near 0 degree, this application is implemented as searching and adjusting the output frequency of said radio frequency generator over the preset frequency range, reducing the difference between said operating frequency and the resonant frequency of said complex impedance to be less than a pre-set threshold.

As a further step the inter-tern spacing of the insulated heating coil is adjusted to have the resonant frequency of said inductance of the insulated heating coil and the expected WPC combined to be in said operating frequency range, wherein the expected WPC is the parasitic capacitance when the distance from the wall of the tubular organ to the catheter is within a preset range. With the adjustment the sensitivity of detection and control is improved to achieve better treatment effect.

This application recorded many technical features, dispersed in different technical schemes. It would be unreasonably long and tedious to list all possible combinations of these technical features. To avoid this problem, the technical features disclosed in this application can be freely combined to form new technical schemes. These technical solutions shall be considered to be disclosed in this application, unless a combination of these technical features is not technically feasible. For example, if an embodiment discloses feature A+B+C, and discloses feature A+B+D+E in another embodiment, and feature C and D are equivalent technical measures with the same effect, technically only one of them suffices and there is no need to include both, feature E could be combined with feature C, then, the combination A+B+C+D should not be considered as a part of this disclosure because its infeasibility, while the combination A+B+C+E shall be considered as a part of the disclosure.

**Description of Drawings**

FIG. 1 is a diagram of the radio frequency thermal ablation system based on the first embodiment of this application;

FIG. 2 is an overall illustration of the radio frequency thermal ablation catheter;

FIG. 3 is an illustration of insulated heating coil based on the first embodiment of this application;

FIG. 4 is a cross-section of the far-end of the radio frequency thermal ablation catheter based on the exemplary A of the first embodiment of the application;

FIG. 5 is an illustration when the wall of tubular organ is close to the radio frequency thermal ablation catheter based on the exemplary A of the first embodiment of the application;

FIG. 6 shows the WPC formed between the insulated heating coil and the tubular organ wall based on the exemplary A of the first embodiment of the application;

FIG. 7 shows a trajectory of the complex impedance of the exemplary insulated heating coil caused by the change of WPC during the thermal ablation treatment process based on the exemplary A of the first embodiment of the application;

FIG. 8 is a low frequency sampling method on voltage and current of the insulated heating coil based on the exemplary A of the first embodiment of the application;

FIG. 9 is an illustration of the impedance characteristic near resonance caused by the inductance of the exemplary insulated heating coil and WPC based on the exemplary A of the first embodiment of the application;

FIG. 10 is a diagram of the radio frequency thermal ablation system based on the first embodiment of this application;

FIG. 11 is a diagram on the calibration mechanism of the circuit parasitic capacitance described in the first embodiment of this application;

FIG. 12 is a flowchart of a control method (not being part of the invention) of a radio frequency thermal ablation system;

FIG. 13 is a flowchart of the process that calibrates the resonance frequency;

FIG. 14 (A) is a flowchart of the whole treatment process using the thermal ablation catheter with flash memory;

FIG. 14 (B) is a flowchart of the whole treatment process using the thermal ablation catheter without flash memory;

wherein,

| | | | |
|---|---|---|---|
| 201- | ablation body | 202- | heating element |
| 203- | tubing | 204- | movable positioner |
| 205- | strain relief ring | 206- | handle button |
| 207- | handle | 208- | inner tube connector |
| 209- | wire | 210- | wire connector |
| 301- | inductive coil1 | 302- | resistive coil |
| 303- | inductive coil2 | 304- | parallel inductive winding |
| 305- | parallel resistive winding | 401- | LED |
| 402- | catheter tip glue | | |

| | | | |
|---|---|---|---|
| 403- | coil winding tubing | 404- | insulated heating coil |
| 405- | insulated casing | 406- | UV glue |
| 407- | far end sealing tubing | 408- | inner chamber tubing |
| 409- | casing of catheter body | 410- | LED wire |
| 501- | tubular organ shrunk to catheter in thermal ablation | 502- | wall of tubular organ |
| 503- | body liquid in tubular organ | 602- | WPC |
| 701- | the impedance of the exemplary insulated heating coil in complex space when treatment starts | | |
| 702- | the inductance of the exemplary insulated heating coil resonates with WPC to be resistive after treatment | | |
| 801- | the target control point of the impedance of exemplary insulated heating coil | | |

## Detailed Description of the Invention

Further technical details of the present invention are disclosed in the description given hereinafter. However, it should be understood that the detailed description and specific examples, while indicating preferred embodiments of the invention, are given by way of illustration only, since various changes and modifications within the scope of the invention will become apparent to those skilled in the art from this detailed description.

Explanation of some terms:

Ablation: Change property of human tissue by applying energy or material to achieve treatment effects.
Thermal ablation: ablation technique by applying heat.
Radio frequency generator: an electronic instrument that generates and controls high frequency signals. When used in ablation, the generator applies radio frequency power to the ablation catheter to achieve ablation treatment effects.
Complex impedance: the impedance calculated from the phase difference between voltage and current, including the real part and the imaginary part.
Tumescent anesthesia: anesthetized fluid is injected into the patient's body, causing swelling and compression of the organs while forming anesthesia, and assisting in the ablation of blood vessels.
EEPROM : Electrically Erasable Programmable read only memory.

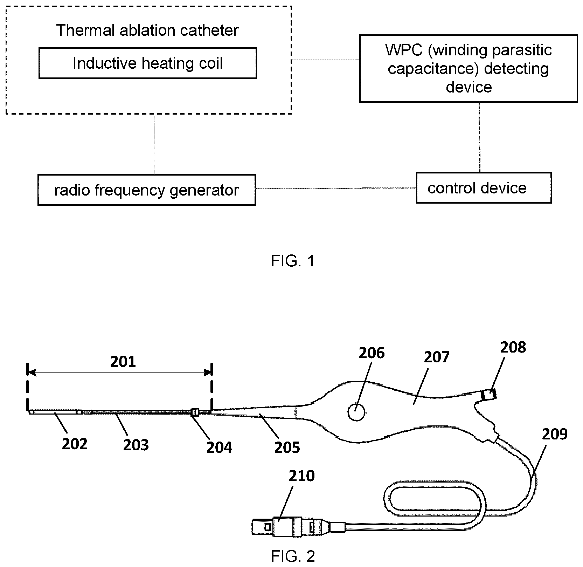

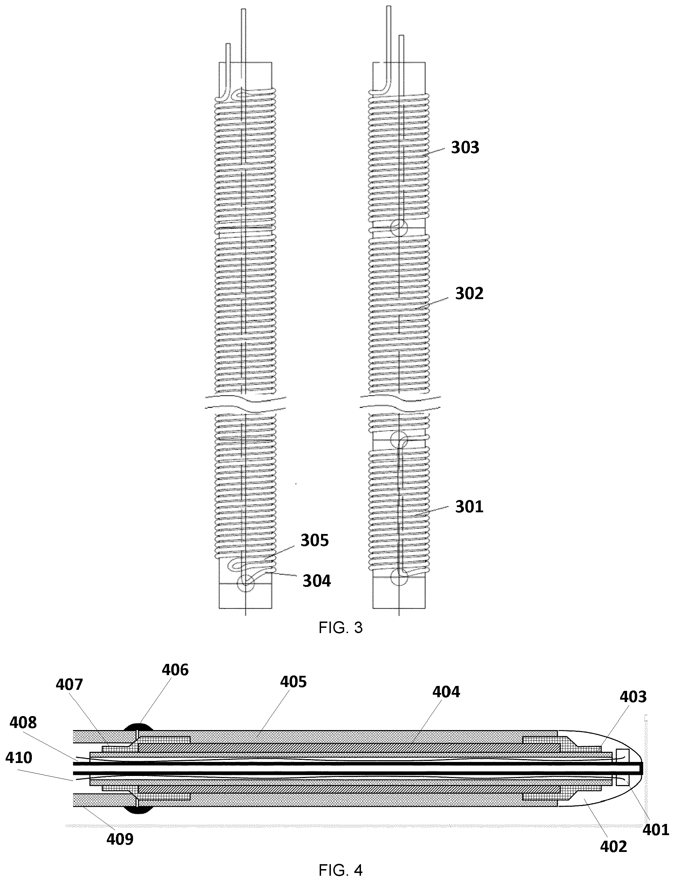

In order to clarify the purpose, technical solutions, and advantages of the present application, the implementation method of the present application will be described in further detail below in conjunction with the accompanying drawings.
At present, vascular thermal ablation technology generally relies on adjusting the heating time at a certain temperature to control the degree of vessel shrinkage. Therefore, the current treatment devices on the market generally control two parameters: heating temperature and heating time. These two are indirectly related to the parameters that need to be controlled during treatment, such as the degree of tubular wall contraction or the degree of body fluid coagulation. Simply put, the longer the heating time, the higher the degree of wall shrinkage of the tubular organ. However, because of the different shapes and sizes of organs, heating time and temperature cannot uniquely determine the degree of tubular wall contraction or body fluid condensation. Empirically controlling the heating temperature and time can lead to differences in therapeutic effects or even out of control. For example, when the heating temperature and time are controlled, if the tube wall shrinks faster than expected, continued heating may cause damage and carbonization of the tubular organ, or make the ablation catheter stick to the inner wall of the tubular organ and cannot be pulled out, causing medical accidents; and when the heating time is too short It will lead to inadequate treatment, incomplete closure of blood vessels, failure to accomplish the purpose of surgery. Moreover, the blood vessel's own condition and surrounding environment are different and variable during the treatment. For example, although the surgeons will try to expel the blood in the blood vessel to be treated through pressing and tumescent anesthesia, there still is certain amount of blood left in the blood vessel, surrounding the heating element, forming an additional load and cooling the heating element. Obviously, if there is extra blood residue, it will take longer to heat to obtain the same effect. For example, different blood vessel segments of different patients or different blood vessel segments of the same patient may present varied sizes and thicknesses. A fixed heating time control cannot accommodate the differences. This eventually leads to either inadequate treatment, or excessive heating that causes tissues to stick to the surface of the thermal ablation catheter. The root cause of these problems is that there are more variables other than temperature and heating time determine the treatment outcome. The essential parameters that need to be monitored and controlled during treatment are the distance from the wall of the tubular organ to the surface of the thermal ablation catheter and the degree of coagulation of the body fluid around the catheter.
Therefore, to address the needs, the first embodiment of this application involves a radio frequency thermal ablation system, the structure of which is shown in FIG. 1.
Specifically, as shown in FIG. 1, the radio frequency thermal ablation system comprises a thermal ablation catheter and a radio frequency generator, wherein the thermal ablation catheter comprises an insulated inductive heating coil. The radio frequency generator outputs radio frequency power to the insulated heating coil for heating. In one embodiment, the inductance is used to configure the frequency operating point during detection to enhance the sensitivity of detecting parasitic capacitance between winding turns.
In one embodiment of the invention, as shown by the overall diagram of the exemplary radio frequency thermal ablation catheter in FIG. 2, it comprises a strain relief ring 205 and an ablation body 201 as an effective treatment segment. The ablation body 201 comprises a heating element 202, a tubing 203 and a movable positioner 204, wherein the heating element 202 is a closed, insulated inductive coil connected to the tubing 203. The movable positioner 204 is installed on the tubing 203. The insulated heating coil prevents electrical current from flowing into human tissue. It shall be understood that the connection between the insulated inductive heating coil and the tubing 203 in this embodiment can be a fixed connection or can be any other reliable forms of connection. Furthermore, the exemplary radio frequency thermal ablation can also comprise a strain relief ring 205, a handle button 206, a handle 207, an inner tube connector 208, a wire 209, and a wire connector 210 etc. In some embodiments, the exemplary radio frequency thermal ablation catheter can be a modified and improved design of any known radio frequency thermal ablation catheter.
Optionally, the insulated heating coil with inductance characteristics is a weakly inductive coil. The weakly inductive insulated heating coil can be realized in various ways. In one embodiment of the invention, as shown in the right half of FIG. 3, the insulated heating coil can comprise an inductive coil 302 consisting of a first part of two overlapping helixes, an inductive coil 301 consisting of a second part of the single helix, and an inductive coil 302 consisting of the third part of the single helix. This centralized winding makes the distribution of coil inductance more concentrated, and can focus the sensing to a specific segment during heating. Compared with the winding in the left half of FIG. 3 (parallel inductive winding 304 + parallel resistive winding 305), the inductance distribution of the latter is relatively spread, basically covering the whole heating element with less selectivity. In some embodiments, the number and arrangement of inductive and resistive windings may be set according to specific needs on sensing selectivity.

Furthermore, the radio frequency thermal ablation system comprises a WPC detecting device that monitors the change of the WPC during the heating process. Then the shrinkage of the wall of the tubular organ and the coagulation of surrounding body fluid can be estimated. The WPC detecting device takes advantage of the resonance of the insulated heating coil and WPC, locates the operating frequency near the resonance frequency to boost sensitivity. The WPC detecting device then calculates WPC value through the phase difference between voltage and current applied on the winding. For example, because the insulated heating coil model near the operating frequency can be considered as an equivalent RLC circuit, and the phase difference between its voltage and current is PH, the equivalent capacitance value can be calculated with the following equation:

$$\tan(PH) = （XL - XC）/ R \qquad\qquad （1）$$

wherein, XL represents inductive impedance, XL=2rrfL, XC represents capacitive impedance, XC=1/(2 $\pi$ fC), f represents operating frequency, R represents equivalent resistance. Therefore, when L is known, the equivalent capacitance can be obtained by detecting the phase. The corresponding relationship between WPC value with the degree of tubular wall shrinkage and coagulation of surrounding body fluid can be calibrated in advance with experiments. At the resonant frequency, $\Delta$ PH/$\Delta$f is maximal and the detection sensitivity is the highest. The detection sensitivity can therefore be improved by setting a preset L value to configure the frequency operating point of detecting the WPC to be near the resonant point. The WPC detecting device may be a separate component or may be combined with other components, for example, it can be a part of the radio frequency generator by combination.

Optionally, the causes for the change in the complex impedance of the insulated heating coil of the thermal ablation catheter covered in this application comprise one or plural following factors: ① the tubular organ wall being thermally ablated shrinks and its wall moves close to the ablation catheter; ② the body fluid outside the thermal ablation catheter gradually coagulates during the heating process. Based on these causes, the WPC can be formed by the capacitance between the tubular organ wall or coagulated body fluid and aforesaid exemplary inductive winding as the tubular organ shrinks and the body liquid coagulates. The WPC gradually increases to resonate with the inductance. In this process, the complex impedance of the insulated heating coil experiences a change from being inductive to resistive on the complex plane. Therefore, the WPC detecting device can estimate and control the degree of treatment during the heating process by monitoring the change of the process.

Optionally, the inductance of the insulated heating coil can be preset as required. In one embodiment of the invention, the inductance of the insulated inductive heating coil can be preset by the following formula (2):

$$L_{loop} \approx N^2 \mu_0 \mu_r \left(\frac{D}{2}\right) \cdot \left(\ln\left(\frac{8 \cdot D}{d}\right) - 2\right) \qquad （2）$$

wherein, N represents the number of turns of the coil, D represents the diameter of the coil, d represents the diameter of the wire, $\mu_0$ represents the permeability of vacuum, $\mu_r$ represents the relative permeability, which is close to 1 in the body fluid. Therefore, the inductance value can be controlled by controlling the conductor diameter d and the gap between coil windings. At the same time, without considering the external medium, the capacitance between the coil turns can be estimated by the following formula (3):

$$C_L = \frac{\varepsilon_0 \pi^2 D}{(N-1)\ln\left[\frac{p}{d} + \sqrt{\left(\frac{p}{d}\right)^2 - 1}\right]} \qquad （3）$$

wherein, $\varepsilon_0$ represents dielectric constant, p represents the distance between the coil, D represents the diameter of the coil, d represents the diameter of the wire, N represents the number of turns of the coil. Therefore, the larger the distance between the coil turns, the smaller the WPC. This principle shows the range of the WPC can be controlled by adjusting the winding parameters.

Furthermore, in order to improve the sensitivity and achieve better monitoring effect, the inter-turn spacing of the heating coil can satisfy the following condition: the resonance frequency caused by the inductance of said heating coil and expected WPC is within said frequency range, wherein said expected WPC is the inter-turn parasitic capacitance of said heating coil when the distance between blood vessel tissue and said thermal ablation catheter is within a pre-set range.

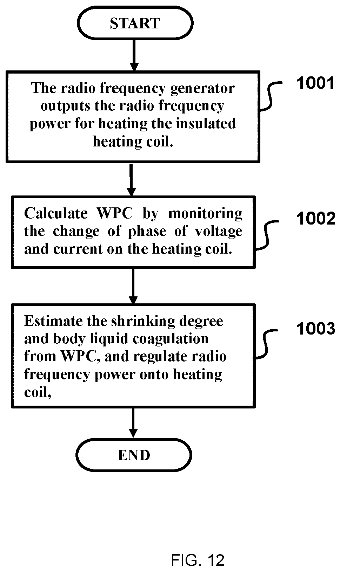

Optionally, the radio frequency thermal ablation system further comprises flash memory for storing pre-calibrated resonant frequency that is readable as the target resonant frequency to said control device. For example, during the calibration of the resonant frequency, a common average value can be used, or each thermal ablation catheter can be calibrated separately and finally stored in the memory. FIG. 12 shows an embodiment with calibration process. In the calibration process, the actual resonant frequency when the wall of the tubular organ reaches the catheter is stored as the target resonance frequency $f_r$ for use by the control device after the calibration.

Varies types of the memory can be used to store the calibration data. For example, it could be EEPROM, or it could be FLASH memory, etc.

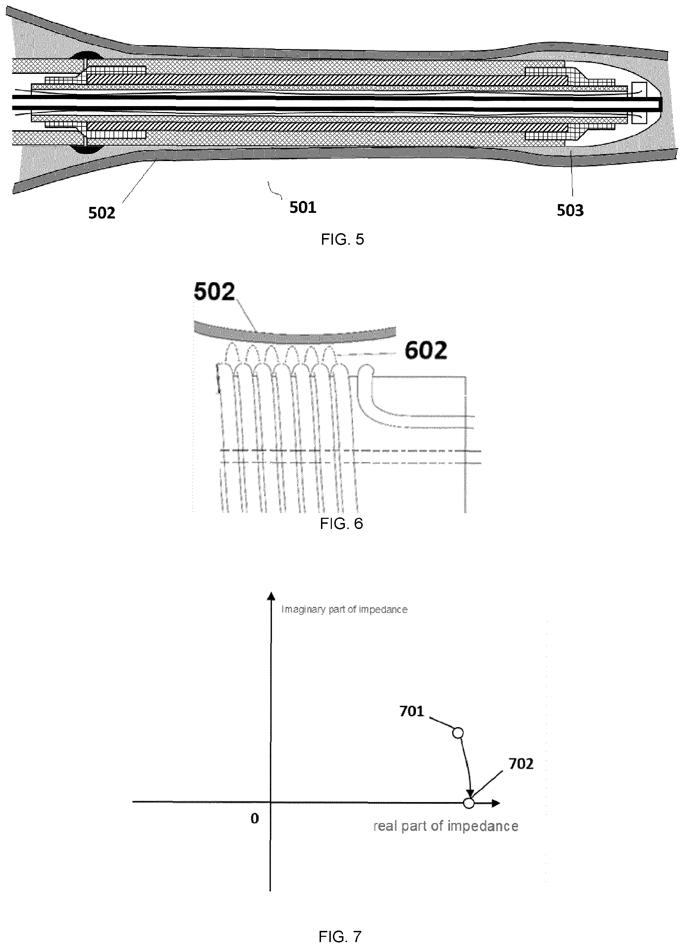

In order to better understand the above process of "monitoring the change of the WPC between the insulated heating coil turns during heating process", we explain with example A. The details included in example A are for the convenience of understanding and are not intended to limit the scope of protection of this application. In example A, a cross-section of the far-end of the radio frequency thermal ablation catheter is shown in FIG. 4. The exemplary thermal ablation catheter comprises a LED 401, catheter tip glue 402, a coil winding tubing 403, an insulated heating coil 404, an insulated casing 405, UV glue 406, a far-end sealing tubing 407, an inner chamber tubing 408, a casing of catheter body 409 and LED wires 410. FIG. 4 shows one possible implementation of the closed and the insulated heating coil, in which the coil current does not flow into the surrounding tissues. As shown in FIG. 5, when the exemplary thermal ablation catheter is used in thermal ablation, the wall of the tubular organ shrinks and approaches the surface of the insulated heating coil. Meanwhile, the body liquid such as blood in the tubular organ 503 outside the insulated heating coil gradually coagulates. Furthermore, as shown in FIG 6, the WPC 602 is formed by the tubular organ wall 601, the coagulated body fluid, and the exemplary inductive winding of the insulated heating coil. Therefore, the shrinkage of the tubular organ wall and/or body fluid coagulation can be deduced with the variation of the WPC 602. The insulated casing 405 is between insulated heating coil 404 and the wall of the tubular organ (502 or 602) and is not shown in Fig 6. The thinner the insulated casing 405, the less effect it has on WPC. Therefore, ultra-thin FEP tubes with wall thickness less than 0.01 mm are adopted to ensure the sensitivity of complex impedance detection, as well as insulation and sealing. As the tubular organ shrinks and the body liquid coagulates, the WPC resonates with the insulated heating coil. Moreover, the phase change rate of complex impedance near the resonant point is higher and the detection is more sensitive. In the above-mentioned process, on the complex plane the impedance trajectory of the exemplary insulated heating coil is shown in Fig 7. In the complex space the impedance of the exemplary insulated heating coil is at position 701 when the treatment starts, and as the treatment finishes, the resonant impedance moves to a resistive position 702.

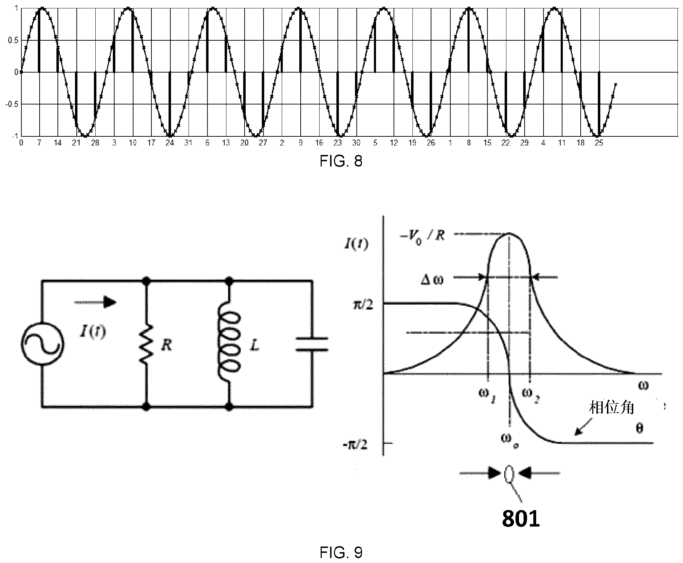

Optionally, said WPC detecting device monitors the change of WPC between insulated heating coil by detecting the voltage and current vectors on the heating coil. A possible embodiment is to sample 32 points of the current and voltage waveform of the insulation heating winding with a fixed phase, which can be distributed over the period at equal intervals, or to assemble sampling signals of one period by sampling in several adjacent periods with varied phases. As shown in FIG 8, if the signal with frequency f needs to generate 32 samples for each period, the conventional method is to sample at 32f. When the signal is narrow band the oversampling can be replaced with a sampling rate of 32f/7, with the sampling rate reduced by 7 times. In this method the samples of one period is expanded to 7 periods, generating 32 samples from S1 to S32. After these 32 samples are collected, their sequence needs to be sorted and re-assembled according to their phases to waveform equivalent to the original signal. The advantage is reduced sampling rate and hardware cost. In the method the sampling rate varies with the master clock. Hence the relative sampling rate remains unchanged and synchronized with the radio frequency output signal when the output frequency changes.

In the premise of synchronous sampling, 32 voltages and currents are obtained. The phase difference is calculated according to the following formula:

$$PH = atan\frac{\sum_{k=1}^{32}(v_k \times s_k)}{\sum_{k=1}^{32}(i_k \times c_k)} \qquad (4)$$

Wherein, Sk is a square wave with the same frequency with the radio frequency signal and with phase 0. When k=1~16, Sk=1; when k=17~32, Sk=-1. Ck is a square wave with the same frequency and with phase 90 degrees. When k=1~8, Ck=1; when k=9~24, Ck=-1; when k=25~32, Ck=1. With this method, the phase difference PH can be computed in real time by using the sampled voltage and current signals in one to several signal cycles, and then the WPC can be estimated.

Furthermore, the radio frequency thermal ablation system comprises a control device. The control device regulates the radio frequency power delivered into the insulated heating coil, based on the complex impedance variation detected by the WPC detecting device. For example, when the complex impedance is close to the pre-set value, the control device assumes that the tubular organ wall is close to the ablation catheter, and it reduces the power to avoid overheating and make the treatment smooth and accurate. The control device can be a separate component or it can be combined with other components. For example, it can be integrated with the WPC detecting device and the radio frequency generator.

Optionally, the control device is also used to calculate the complex impedance according to the voltage vector and current vector on the insulated heating coil detected by WPC detecting device. The control device computes the phase of the complex impedance and obtains the WPC of the insulated heating coil. Then the distance of tubular wall and the degree of coagulation of surrounding body fluid can be detected. If the WPC of the insulated heating coil is within a specified range, the radio frequency generator is controlled to reduce the power output, thus reduce the shrinkage of the wall of the organs. When the distance of tubular wall or the body fluid coagulation estimated with WPC reaches a pre-set threshold, the controller indicates that the target is reached and ceases the delivery of radio frequency power into the insulated heating coil.

Optionally, the control device is also used to search and adjust the frequency of the radio frequency power output from said radio frequency generator in a preset frequency range, so that the difference between said frequency and the resonant frequency of said complex impedance is less than a preset threshold. This is because the sensing circuit performs with better sensitivity when the phase is near 0 degree. In one embodiment of the invention, the operating frequency shall be close, if not equal to the resonance frequency of the complex impedance. Because the resonance frequency changes with the WPC, perfect equalization is difficult. A more practical method is to ensure the difference between the operating frequency and the resonant frequency of said complex impedance to be less than a preset threshold. For example, in a practical application dynamic tracking control can stably keep the difference less than 2 degrees. With the output power and heating, the wall of the tubular organ shrinks and the resonant frequency moves. The output frequency can follow the movement to obtain the best sensitivity until the WPC enters the preset interval and the controller decreases power output until it is zero.

Optionally, the operating frequency range comprises the frequency range in which the phase of the complex impedance of the insulated heating coil shifts from - 90 degrees to 90 degrees after said parasitic capacitance is cancelled. In this frequency range the inductance of the designed insulated heating coil resonates with the WPC. The WPC and the inductance of the insulated heating coil are relatively weak. In order to exclude the influence of the parasitic capacitance of the instrument circuit and wire, the parasitic impedance other than the insulated heating coil can be calibrated in advance, and the parasitic impedance can be cancelled in real-time WPC monitoring with software.

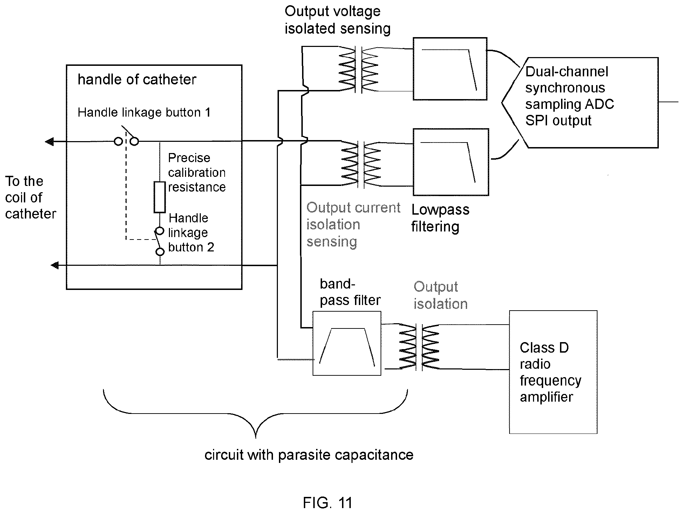

The WPC and inductance of the insulated heating coil are relatively weak, the parasite capacitance (circuit capacitance) of the instrument circuit and wire is generally greater than the WPC. The capacitance of the circuit directly affects the measured frequency characteristics of the complex impedance curve as shown in FIG 8, which causes a phase drift over the operating frequency range. Consequently the criterion using 0 degree as the target is no longer valid. A coupled switch in the catheter handle as shown in Fig 11 provides a mechanism to effectively offset circuit capacitance. A precision calibration resistor is embedded in the handle. The coupling switch controls both the output switch and the calibration switch. When the coupling switch 1 is disconnected, the coupling switch 2 is closed, and the precision calibration resistor is connected to the power output circuit, and the catheter coil is disconnected at the same time. The sampling circuit measures the precision calibration resistance with known resistance and the parasite capacitance of the circuit with unknown value. When the coupling switch 1 is closed, the coupling switch 2 is disconnected, and the parasitic capacitance of the circuit is connected to the power output circuit together with the catheter coil. the DSP thus cancels the influence of the parasitic capacitance of the circuit with software.

Still in the above example A, FIG 9 shows the impedance characteristics near the resonance caused by the inductance of the insulated heating coil and WPC. The control device can fine-tune the operating frequency of the radio frequency generator according to the test results generated by the WPC detecting device. By doing so the control device maintains the frequency operating point at the target control point 801 of the impedance of exemplary insulated heating coil as shown in FIG 8, until the operating frequency is close to the initial operating frequency of the insulated heating coil.

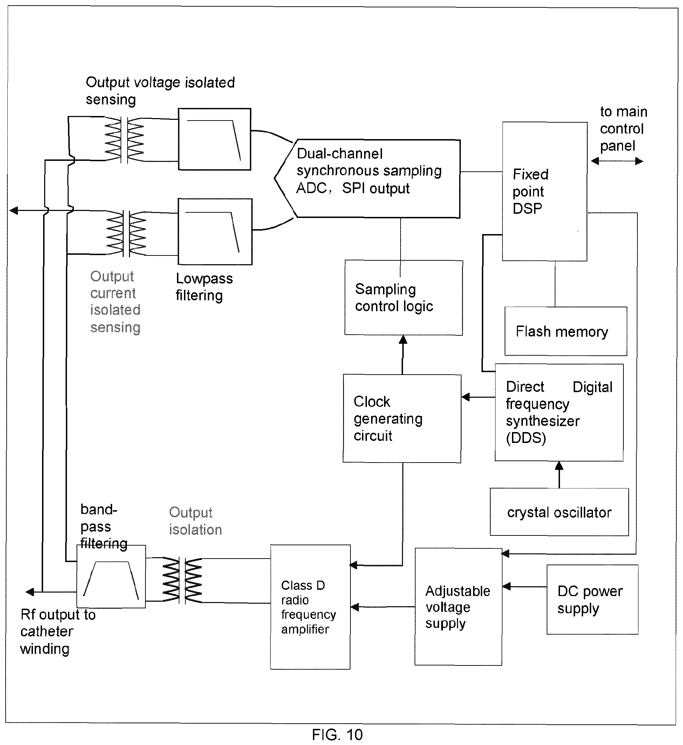

In an embodiment of the present invention, FIG. 10 is a diagram of the radio frequency thermal ablation system using the exemplary radio frequency generator circuitry. The components and details in the block diagram of the exemplary radio frequency generator are only one of the many possible implementations. The present invention is not limited to what has been particularly shown and described hereinabove. Variations on hardware or software may be made in the construction and relation of parts without departing from the scope of the invention described herein.

A control method of a radio frequency thermal ablation system (not being part of the invention) is described in the follwoing. The radio frequency thermal ablation system comprises a thermal ablation catheter and a radio frequency generator, the thermal ablation catheter comprises an inductive insulated heating coil. The radio frequency generator outputs radio frequency power to the insulated heating coil for heating.

The inductance is designed to configure the operating frequency when detecting the parasitic capacitance between winding turns, and enhance the detecting sensitivity.

The radio frequency thermal ablation system referred to in this embodiment is the radio frequency thermal ablation system of the first embodiment, and the technical details of the radio frequency thermal ablation system of the first embodiment may be applied.

FIG. 11 is a process diagram of the control method of above-mentioned radio frequency thermal ablation system. As shown in FIG. 10, the control method of the radio frequency thermal ablation system can specifically comprise the following steps:

In step 1001, the radio frequency generator outputs the radio frequency power for heating to the insulated heating coil.

Subsequently, in step 1002, the radio frequency generator monitors the change of WPC caused by the tubular organ wall approaching the insulated heating windings and/or the coagulation of body fluid during the heating process. Specifically, The WPC can be calculated by monitoring the change of phase of voltage and current on the heating coil.

Optionally, the causes for the change in the complex impedance of the insulated heating coil of the thermal ablation catheter covered in this application comprise one or a combination of the following: ① the wall of the tubular organ shrinks during thermal ablation, and moves towards the ablation catheter; ② the body fluid outside the thermal ablation catheter gradually coagulates during the heating process.

Optionally, after step 1002, the control method further comprises the following steps: in the pre-set operating frequency range, search and adjust the output frequency from said radio frequency generator, reduce the difference between said operating frequency and the resonant frequency of said complex impedance to be less than a pre-set threshold.

Optionally, after said parasitic capacitance is cancelled, the operating frequency range includes the frequency range in which the phase of the complex impedance of the insulated heating coil shifts from -90 degrees to 90 degrees. In order to exclude the influence of the parasitic capacitance of the instrument circuit and wires, the parasitic impedance other than the insulated heating coil can be calibrated in advance during production or use, and then offset with software algorithm when monitor the WPC in real-time. For example, the parasitic capacitance of the circuit calibrated during production is Cp, and Cp is in parallel with the insulated heating coil in the calculation model. The current flowing through Cp is:

$$\vec{I_p} = \frac{\vec{V_o}}{1/_{jwC_p}} \qquad (5)$$

wherein, Vo represents output voltage, Ip represents the current of the parasitic capacitance, Io represents total output current, Then the actual current flowing through the insulated heating coil Ih is:

$$\vec{I_h} = \vec{I_o} - \frac{\vec{V_o}}{1/_{jwC_p}} \qquad (6)$$

Therefore, The WPC after offsetting the influence of parasitic capacitance of the circuit can be calculated from the range of 90 degrees to -90 degrees according to the relationship between Ih and Vo.

Then, in step 1003, the control device that controls the amount of radio frequency power output from said radio frequency generator to said heating coil according to the change of WPC (In other words, the shrink of the organ wall and/or body fluid coagulation can be deduced from the variation of the WPC). The control algorithm can adopt the common PID (proportional integral difference) control method and so on, and obtain the continuous and smooth control effect based on the treatment parameters like the shrinking degree of the organ wall and/or body fluid coagulation.

Optionally, the process described in above steps 1002-1003 on monitoring the change of complex impedance of the insulated heating coil, calculating WPC of the heating coil based on the detected change of the phase of the complex impedance, deducing the shrinking degree of the organ wall and/or body fluid coagulation, and controlling the radio frequency power output to the insulation heating coil, can further comprise the following steps from i to iii:

i . Obtain the vector voltage and current on the insulation heating coil;

ii. Calculate the complex impedance of the insulated heating coil according to vector voltage and current and calculate the phase of the complex impedance;

iii. Adjust the radio frequency according to the WPC calculated from the phase of the complex impedance. If said phase is within a pre-set range, said control method stops said radio frequency generator from outputting radio frequency power.

Optionally, the inter-turn spacing of the heating coil satisfies the following condition: the resonance frequency caused by the inductance of said heating coil and expected WPC is within said frequency range, wherein said expected WPC is the inter-turn parasitic capacitance of said heating coil when the distance between blood vessel tissue and said thermal ablation catheter is within a pre-set range.

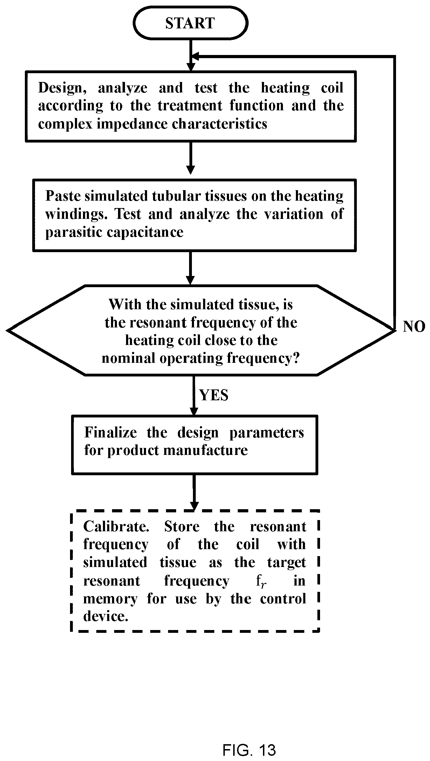

Optionally, resonant frequency can be pre-calibrated. Because of the corresponding relationship between WPC and resonant frequency, the resonant frequency can be indirectly used as a target for detection and control. FIG. 13 shows a test calibration process for an embodiment. After the calibration, the resonant frequency when the actual tubular organ is shrunk to the catheter is stored in memory as the target resonant frequency $f_r$ for use by the control device.

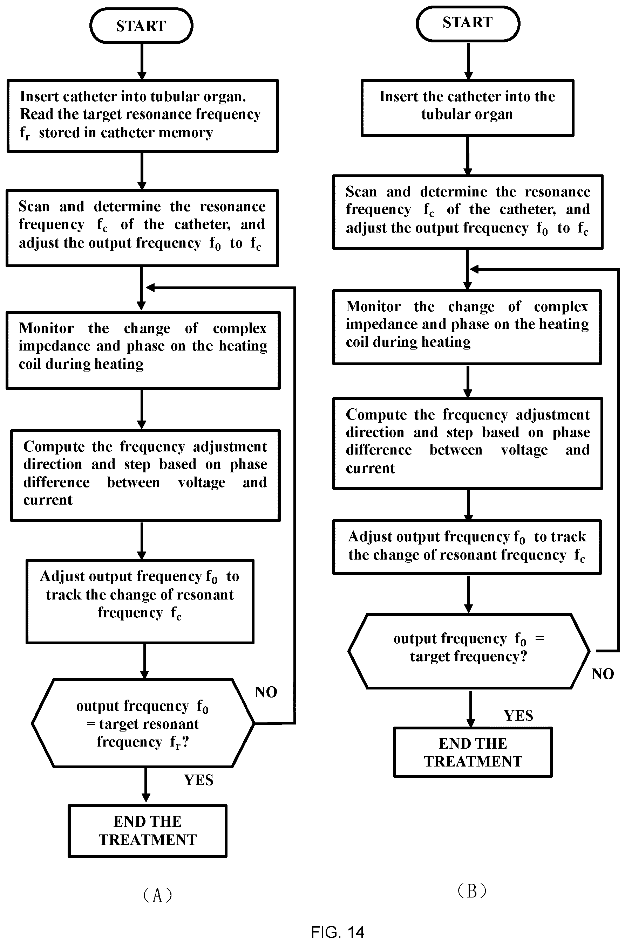

Two cases are considered for the thermal ablation catheter with and without memory, respectively. The output frequency of the radio frequency generator was adjusted according to Figure 14 (A) and Figure 14 (B) respectively to control the entire treatment process. FIG. 14 (A) is the case of the thermal ablation catheter with memory. The target output frequency of said radio frequency generator during treatment is the target resonant frequency fr stored during calibration, fr is the resonant frequency when the actual tubular organ is shrunk to the catheter. FIG. 14 (B) illustrates the process of the catheter without memory. A unified target frequency is used as the target frequency of the radio frequency generator during treatment.

It should be noted that in the application of this patent, relational terms such as first and second are used only to distinguish one entity or operation from another and do not necessarily require or imply any such actual order or sequence between these entities or operations. Also, the term "comprising" and "comprise" or any of its other variants is intended to cover a non-exclusive inclusiveness, which means a process, method, article or equipment with a series of elements, not only includes those specified elements, but also other elements that are not explicitly listed, plus the intrinsic elements of this process, method, article or equipment. In the absence of additional specifications, the elements defined by the statement "comprise a" do not exclude the existence of additional identical elements in the process, method, article, or device that includes that element. Any references in this application to an act performed according to an element mean the act is performed at least according to that element, which includes two cases: the act is performed in accordance with that element alone, and the act is performed in according to that element and other elements. 'Plural' means 2 or more than 2.

The invention is defined by the independent claim. It should be understood that the foregoing are only preferred embodiments of this invention and are not intended to limit the scope of protection of this application.

In some cases, the actions or steps recorded in the claims may be performed in a different sequence and still achieve desired results. In addition, the processes illustrated in the figures do not necessarily require a specific sequence or continuity to achieve desired results. Multitasking and parallel processing are also possible or may be beneficial in some embodiments.

## Claims

1. A radio frequency thermal ablation system, comprising:

   a thermal ablation catheter that comprises an inductive insulated heating coil (404), wherein the inductance is designed to configure the operating frequency when detecting a parasitic capacitance between winding turns, and enhance the detecting sensitivity;
   a radio frequency generator that supplies radio frequency power to heat said heating coil (404);
   a winding parasitic capacitance WPC detecting device that monitors the change of the WPC (602) between the insulation heating windings caused by the tubular organ wall (502) approaching the insulated heating windings and/or the coagulation of body fluid during the heating process, wherein said WPC detecting device monitors the change of WPC (602) between said heating coil winding turns by detecting the voltage and current vectors on the heating coil (404);
   a control device that controls the amount of radio frequency power output from said radio frequency generator to said heating coil (404) according to the change of said WPC (602) between the windings detected by said WPC detecting device.

2. The radio frequency thermal ablation system of Claim 1, wherein the control device further calculates the parasitic capacitance between said heating coil winding turns based on the vector voltage and current of the heating coil (404) detected by the WPC detecting device, and said control device extracts the phase of the combined impedance of the parasitic capacitance between the heating coil winding turns and said coil inductance; if the phase is in a preset interval, said control device stops outputting radio frequency power to the heating coil (404).

3. The radio frequency thermal ablation system of Claim 2, wherein the output frequency of said control device is continuously adjustable, and said control device searches and adjusts the frequency of the radio frequency power output from said radio frequency generator in a preset frequency range, so that the difference between said frequency and the resonant frequency of said complex impedance is less than a preset threshold.

4. The radio frequency thermal ablation system of Claim 3, wherein the operating frequency range includes the frequency range in which the phase of the complex impedance of the heating coil shifts from -90 degrees to 90 degrees after said parasitic capacitance is cancelled.

5. The radio frequency thermal ablation system of Claim 3, wherein the inter-turn spacing of the heating coil (404) satisfies the following condition: the resonance frequency caused by the inductance of said heating coil (404) and expected WPC (602) is within said frequency range, wherein said expected WPC (602) is the inter-turn parasitic capacitance of said heating coil (404) when the distance between blood vessel tissue and said thermal ablation catheter is within a pre-set range.

6. The radio frequency thermal ablation system of Claim 5 , further comprising flash memory for storing pre-calibrated resonant frequency that is readable as the target resonant frequency to said control device.


**Patentansprüche**

1. Hochfrequenzwärmeablationssystem, umfassend:

   einen Wärmeablationskatheter, der eine induktive isolierte Heizspule (404) umfasst, wobei die Induktivität ausgelegt ist, die Betriebsfrequenz zu konfigurieren, wenn eine parasitäre Kapazität zwischen Wicklungswindungen erfasst wird, und die Erfassungsempfindlichkeit zu verbessern;
   einen Hochfrequenzgenerator, der Hochfrequenzleistung zum Erhitzen der Heizspule (404) zuführt;
   eine Erfassungsvorrichtung für parasitäre Wicklungskapazität, WPC (Winding Parasitic Capacitance), die die Änderung der WPC (602) zwischen den Isolierheizwicklungen überwacht, die durch Annäherung der Hohlorganwand (502) an die isolierten Heizwicklungen und/oder Koagulation von Körperflüssigkeit während des Erhitzungsprozesses verursacht wird, wobei die WPC-Erfassungsvorrichtung die Änderung von WPC (602) zwischen Heizspulenwicklungswindungen durch Erfassen der Spannungs- und Stromvektoren an der Heizspule (404) überwacht;
   eine Steuervorrichtung, die die Menge an Hochfrequenzleistung, die von dem Hochfrequenzgenerator an die Heizspule (404) ausgegeben wird, entsprechend der Änderung der WPC (602) zwischen den Wicklungen, die von der WPC-Erfassungsvorrichtung erfasst wird, steuert.

2. Hochfrequenzwärmeablationssystem nach Anspruch 1, wobei die Steuervorrichtung weiter die parasitäre Kapazität zwischen den Heizspulenwicklungswindungen basierend auf der Vektorspannung und dem Vektorstrom der Heizspule (404) berechnet, die von der WPC-Erfassungsvorrichtung erfasst werden, und die Steuervorrichtung die Phase der kombinierten Impedanz der parasitären Kapazität zwischen den Heizspulenwicklungswindungen und der Spuleninduktivität extrahiert; wenn die Phase in einem voreingestellten Intervall ist, die Steuervorrichtung Ausgeben von Hochfrequenzleistung an die Heizspule (404) stoppt.

3. Hochfrequenzwärmeablationssystem nach Anspruch 2, wobei die Ausgegebene Frequenz der Steuervorrichtung kontinuierlich einstellbar ist und die Steuervorrichtung die Frequenz der Hochfrequenzleistung, die von dem Hochfrequenzgenerator ausgegeben wird, in einem voreingestellten Frequenzbereich sucht und einstellt, sodass der Unterschied zwischen der Frequenz und der Resonanzfrequenz der komplexen Impedanz kleiner als ein voreingestellter Schwellenwert ist.

4. Hochfrequenzwärmeablationssystem nach Anspruch 3, wobei der Betriebsfrequenzbereich den Frequenzbereich enthält, in dem die Phase der komplexen Impedanz der Heizspule -90 Grad bis 90 Grad verschoben ist, nachdem die parasitäre Kapazität aufgehoben worden ist.

5. Hochfrequenzwärmeablationssystem nach Anspruch 3, wobei der Abstand zwischen Windungen der Heizspule (404) die folgende Bedingung erfüllt; die Resonanzfrequenz, die durch die Induktivität der Heizspule (404) und erwartete WPC (602) verursacht wird, ist innerhalb des Frequenzbereichs, wobei die erwartete WPC (602) die parasitäre Kapazität zwischen Windungen der Heizspule (404) ist, wenn der Abstand zwischen Blutgefäßgewebe und dem Wärmeablationskatheter innerhalb eines voreingestellten Bereichs ist.

**6.** Hochfrequenzwärmeablationssystem nach Anspruch 5, weiter umfassend einen Flash-Speicher zum Speichern einer vorab kalibrierten Resonanzfrequenz, die als die Soll-Resonanzfrequenz für die Steuereinheit lesbar ist.

**Revendications**

**1.** Système d'ablation thermique radiofréquence, comprenant :

un cathéter d'ablation thermique qui comprend une bobine de chauffage isolée inductible (404), l'inductance étant conçue pour configurer la fréquence d'opération quand on détecte une capacité électrique parasitaire entre tours de bobinage et augmenter la sensibilité de détection ;
un générateur radiofréquence qui fournit la puissance de radiofréquence pour chauffer ladite bobine de chauffage (404) ;
un dispositif de détection de capacité électrique parasitaire de WPC qui suit le changement de la WPC (602) entre les bobinages de chauffage d'isolation provoqués par la paroi d'organe tubulaire (502) approchant les bobinages de chauffage isolés et/ou la coagulation de fluide corporel durant le processus de chauffage, ledit dispositif de détection de WPC suivant le changement de WPC (602) entre lesdits tours de bobinage de bobine de chauffage en détectant les vecteurs de tension et de courant sur la bobine de chauffage (404) ;
un dispositif de commande qui commande la quantité de sortie de puissance de radiofréquence dudit générateur de radiofréquence à ladite bobine de chauffage (404) selon le changement de ladite WPC (602) entre les bobinages détecté par ledit dispositif de détection de WPC.

**2.** Système d'ablation thermique radiofréquence selon la revendication 1, dans lequel le dispositif de commande calcule en outre la capacité électrique parasitaire ente lesdits tours de bobinage de bobine de chauffage sur la base de la tension et du courant de vecteur de la bobine de chauffage (404) détectés par le dispositif de détection de WPC et ledit dispositif de commande extrait la phase de l'impédance combinée de la capacité électrique parasitaire entre les tours de bobinage de la bobine de chauffage et ladite inductance de bobine ; si la phase est dans un intervalle préréglé, ledit dispositif de commande interrompt la puissance de radiofréquence de sortie à la bobine de chauffage (404).

**3.** Système d'ablation thermique radiofréquence selon la revendication 2, dans lequel la fréquence de sortie dudit dispositif de commande est ajustable en continu et ledit dispositif de commande cherche et ajuste la fréquence de la sortie de puissance de radiofréquence dudit générateur de radiofréquence dans une plage de fréquence préréglée, de sorte que la différence ente ladite fréquence et la fréquence résonante de ladite impédance complexe est inférieure à un seuil préréglé.

**4.** Système d'ablation thermique radiofréquence selon la revendication 3, dans lequel la plage de fréquence d'opération comprend la plage de fréquence dans laquelle la phase de l'impédance complexe de la bobine de chauffage glisse de -90 degrés à 90 degrés après que ladite capacité électrique parasitaire est annulée.

**5.** Système d'ablation thermique radiofréquence selon la revendication 3, dans lequel l'espacement inter-tour de la bobine de chauffage (404) satisfait la condition suivante : la fréquence de résonance provoquée par l'inductance de ladite bobine de chauffage (404) et la WPC (602) attendue se situent dans ladite plage de fréquence, ladite WPC (602) attendue étant la capacité électrique parasitaire inter-tour de ladite bobine de chauffage (404) quand la distance entre le tissu de vaisseau sanguin et ledit cathéter d'ablation thermique est dans une plage pré-réglée.

**6.** Système d'ablation thermique radiofréquence selon la revendication 5, comprenant en outre une mémoire flash pour stocker la fréquence résonante pré-calibrée qui est lisible comme la fréquence résonante cible audit dispositif de commande.

Thermal ablation catheter

Inductive heating coil

WPC (winding parasitic capacitance) detecting device

radio frequency generator

control device

FIG. 1

201

206  207  208

202  203  204  205

209

210

FIG. 2

FIG. 3

FIG. 4

FIG. 5

FIG. 6

FIG. 7

FIG. 8

FIG. 9

FIG. 10

Output voltage
isolated sensing

handle of catheter

Handle linkage button 1

To the
coil of
catheter

Precise
calibration
resistance

Handle
linkage
button 2

Dual-channel
synchronous
sampling ADC
SPI output

Output current   Lowpass
isolation        filtering
sensing

band-        Output
pass filter   isolation

Class D
radio
frequency
amplifier

circuit with parasite capacitance

FIG. 11

```
                        ┌─────────────┐
                        │    START    │
                        └──────┬──────┘
                               │
                               ▼
      ┌─────────────────────────────────┐
      │  The radio frequency generator  │  ⌐  1001
      │  outputs the radio frequency    │
      │  power for heating the insulated│
      │  heating coil.                  │
      └────────────────┬────────────────┘
                       │
                       ▼
      ┌─────────────────────────────────┐
      │  Calculate WPC by monitoring    │  ⌐  1002
      │  the change of phase of voltage │
      │  and current on the heating coil.│
      └────────────────┬────────────────┘
                       │
                       ▼
      ┌─────────────────────────────────┐
      │  Estimate the shrinking degree  │  ⌐  1003
      │  and body liquid coagulation    │
      │  from WPC, and regulate radio   │
      │  frequency power onto heating   │
      │  coil,                          │
      └────────────────┬────────────────┘
                       │
                       ▼
                ┌─────────────┐
                │     END     │
                └─────────────┘
```

FIG. 12

```
                    ┌─────────────┐
                    │    START    │
                    └─────────────┘
                           │
                           ▼
   ┌──────────────────────────────────────────┐
   │ Design, analyze and test the heating coil │
   │ according to the treatment function and the│
   │ complex impedance characteristics          │
   └──────────────────────────────────────────┘
                           │
                           ▼
   ┌──────────────────────────────────────────┐
   │ Paste simulated tubular tissues on the heating│
   │ windings. Test and analyze the variation of  │
   │ parasitic capacitance                        │
   └──────────────────────────────────────────┘
                           │
                           ▼
          With the simulated tissue, is
          the resonant frequency of the          NO
          heating coil close to the
          nominal operating frequency?
                           │ YES
                           ▼
   ┌──────────────────────────────────────────┐
   │ Finalize the design parameters            │
   │ for product manufacture                   │
   └──────────────────────────────────────────┘
                           │
                           ▼
   ┌ ─ ─ ─ ─ ─ ─ ─ ─ ─ ─ ─ ─ ─ ─ ─ ─ ─ ┐
     Calibrate. Store the resonant
   │ frequency of the coil with          │
     simulated tissue as the target
   │ resonant frequency $f_r$ in         │
     memory for use by the control
   │ device.                             │
   └ ─ ─ ─ ─ ─ ─ ─ ─ ─ ─ ─ ─ ─ ─ ─ ─ ─ ┘
```

FIG. 13

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7837677 B
- EP 1906853 A
- US 20170202600 A1
- EP 2662044 A
- US 10357305 B
- US 2017238991 A1
- EP 3093626 A1